# EUROPEAN PATENT APPLICATION

(11) **EP 4 190 373 A1**
(43) Date of publication of application: **07.06.2023**
(21) Application number: 21212037.2
(22) Date of filing: 02.12.2021
(51) Int. Cl.: A61M 1/06

(54) **A BREAST PUMP OR MILK COLLECTION ASSEMBLY**

(71) Applicant: Koninklijke Philips N.V., 5656 AG Eindhoven (NL)
(72) Inventor: DOBRUSSKIN, Christoph, Eindhoven (NL); BOURQUIN, Yannyk Parulian Julian, Eindhoven (NL); WADHWA, Sahil, Eindhoven (NL); WIJNOLTZ, Anna Louise, Eindhoven (NL)
(74) Representative: Philips Intellectual Property & Standards

(57) **Abstract**

A breast pump system comprises a set of modules. There is at least a breast shield module, a pump module, a milk container module and a treatment module. In a breast pump configuration, the breast shield module, the pump module and milk container module are used without the treatment module, whereas in a treatment configuration, one or more, but not all, of the modules used in the breast pump configuration is exchanged for the treatment module.

## Description

### FIELD OF THE INVENTION

This invention relates to breast pumps.

### BACKGROUND OF THE INVENTION

Breast pumps are used by breast feeding women to extract milk from their breast such that the extracted milk can be fed to their babies at a later time.

It is well known that best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

In order to avoid contamination of the pump with milk residue, many breast pumps employ a moveable membrane, known as a diaphragm, between the pump and the expression kit, the membrane forming a barrier against any milk being sucked into the pump. In some breast pumps, the membrane acts furthermore as a massaging element on the nipple, areola and/or breast of the user. The membrane touches parts of the user's breast during the use of the breast pump. There is movement away from the breast when vacuum is applied and towards the breast when vacuum is released. This stimulates the milk release of the user.

An opening in the chamber between the breast and the membrane guides the milk to a collection container. To prevent milk from flowing back into the chamber, it is known to position a valve between the chamber and the container. The valve furthermore separates the pressure changes in the chamber from the volume of the container.

Mothers that are expressing, or even just breast feeding their babies, can often experience various problems with their breasts, for example nipple cracking, mastitis or candida infection. Often the treatment of these problems requires particular care and attention and even specialist equipment, which is often not readily available.

It would be desirable for a mother to have a system for addressing these problems but without needing significant additional equipment.

### SUMMARY OF THE INVENTION

The invention is defined by the claims.

According to examples in accordance with the invention, there is provided a breast pump system comprising a set of modules, the set of modules comprising:
a breast shield module including at least a breast shield;
a pump module including at least a pump unit;
a milk container module; and
a treatment module,
wherein the breast pump system is configurable in:
   a breast pump configuration with a first set of the modules including the breast shield module, the pump module and milk container module and without the treatment module; and
   a treatment configuration with a second set of the modules, in which one or more, but not all, of the first set of modules is exchanged for the treatment module.

This system can be configured as a breast pump, and if the mother is experiencing pain or other discomfort the system enables this to be addressed by using a treatment function of the system. For this purpose, the system can be reconfigured into a treatment mode. The configuration in the treatment mode shares at least some modules with the configuration in the breast pump mode, so that the overall amount of apparatus needed is reduced.

The system may for example comprise a sensor module, for example for sensing milk flow rate or milk flow volumes, or for sensing physiological data such as vital signs of the mother. A processing module may be provided for processing the sensor module data.

One of the modules also has the function of energy provision for the whole breast pump system. This energy provision may be a separate module or it may be combined with one of the other modules. For example, the energy provision, e.g. by means of a battery, may be part of the pump module. The module with the energy provision may be used in both the breast pump configuration and the treatment configuration.

Similarly, the pump module may be used in both the breast pump configuration and the treatment configuration, for example if the treatment can be implemented using actuation provided by a pump.

The breast pump system is for example for wearing inside a bra, and all modules may fit together as a smooth overall design.

The pump module for example comprises the pump unit and an electronics unit. These two units may be separable (so they could be considered as separate modules) or they may be a single integrated module. The electronics unit controls the operation of the pump, for example implementing a cyclic pressure waveform. There may be a stimulation mode and an expression mode, with different cyclic frequencies.

The electronics unit for example also provides a user interface, and this user interface may be shared between the different configurations of the system. Units may communicate with each other, for example by means of wired or wireless communication. The electric power supply may be transferred from one module to another, for example by wired or wireless (e.g. inductive transfer) means.

The breast shield module may comprise the breast shield and a diaphragm. Again, these two units may be separable (so they could be considered as separate modules) or they may be an single integrated module.

The diaphragm is for example formed of a flexible membrane and comprises a cavity having an open end for receiving a nipple of a user and a closed end, wherein the diaphragm fits over an opening in the breast shield. This provides a compact design. The open end receives the nipple and also functions as the milk outlet (since the other end is closed).

The breast shield and the diaphragm for example form a rear assembly and the pump module is part of a front assembly that fits over the rear assembly. The front assembly further comprises a cover for fitting over the diaphragm to define a sealed space between them. The pump applies an under pressure to the sealed space to implement the breast pump mode.

In one example, the second set of the modules, for the treatment configuration, comprises the pump module and the treatment module without the milk container module and without the breast shield module. Thus, at least the breast shield module and milk container are replaced by a treatment module.

In another example, the second set of the modules, for the treatment configuration, comprises the pump module, the treatment module and the breast shield, without the milk container module and without the diaphragm. Thus, only the diaphragm of the breast shield module, and the milk container, are replaced by a treatment module.

In all examples above, the set of modules may include a treatment control module, wherein the second set of the modules, for the treatment configuration, comprises at least the treatment module and the treatment control module. Thus, the treatment module may be associated with its own controller, separate to the pump controller for the breast pump function.

In one set of examples, the treatment module comprises a massage unit. The massage unit can be operated electrically, pneumatically or mechanically, provided that it results in a mechanical effect. The massage can be exerted onto the breast, the areola and/or the nipple.

In another set of examples, the treatment module comprises a light delivery unit for phototherapy. In this case, the light delivery unit may be for delivering light with a peak intensity at a wavelength in the range 400nm to 470nm (i.e. blue light) or infrared light with a peak intensity at a wavelength in the range 600nm to 800nm.

In another set of examples, the treatment module comprises an RF delivery system or an ultrasound delivery system or a cooling system.

In yet another set of examples, the treatment module comprises a system for delivering topical medication, wherein the set of modules further comprise a topical medication storage container.

The set of modules may further comprise a treatment monitoring sensor module, and the second set of the modules, for the treatment configuration, includes the treatment monitoring sensor module. This may be used to monitor the effectiveness of a delivered treatment.

The treatment monitoring sensor system for example comprises one or more of:
an optical sensor for monitoring skin color, skin hydration, skin oiliness, blood perfusion or heart rate;
an impedance or capacitance sensor for monitoring skin hydration or skin oiliness;
an ultrasound sensor for monitoring skin condition; or
a temperature sensor for measuring skin temperature.

The optical sensor may be implemented by a camera.

The invention also provides a treatment module for applying a treatment to a breast, adapted to be exchangeable with one or more, but not all, of the modules of the first set of the modules of the breast pump system defined above, thereby to result in the second set of modules.

The invention also provides a breast pump module adapted to form one of the first set of modules of the breast pump system defined above, wherein said breast pump module is adapted to be exchangeable with a treatment module, thereby to result in the second set of modules. This is a breast pump module which is swapped for the treatment module.

The invention also provides a breast pump module adapted to form one of the first set of modules of the breast pump system defined above, wherein said breast pump module is adapted to cooperate with a treatment module to enable application of treatment to a breast, when the breast pump system is in the treatment configuration. This is a breast pump module which is used in both configurations (such as a power supply or user interface).

The invention also provides a treatment module for applying a treatment to a breast, wherein the treatment module is adapted to be combined with one or more further modules of a first set of modules, the first set of modules comprising:
a breast shield module including at least a breast shield;
a pump module including at least a pump unit; and
a milk container module,
wherein the combination results in a breast pump system configured in a treatment configuration.

The invention also provides a breast pump module, comprising one of a first set of modules which together define a breast pump configuration of a breast pump system, the first set comprising:
a breast shield module including at least a breast shield;
a pump module including at least a pump unit; and
a milk container module,
wherein the breast pump module is adapted to be exchangeable with a treatment module thereby to result in a second set of modules which together define a treatment configuration of the breast pump system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a wearable breast pump;
Figure 3 shows a breast shield, a diaphragm and a cover over the diaphragm to define a sealed space between them and a milk collection container;
Figure 4 shows one example of a design of the diaphragm;
Figure 5 shows a slightly different design of the diaphragm to Figure 4;
Figure 6 shows a cross section of the assembled breast pump applied to the breast;
Figure 7 shows in exploded view that the breast pump comprises three modular parts;
Figure 8 shows the three modular parts coupled together;
Figure 9 shows two possible disassembly routines;
Figure 10 shows an assembly routine; and
Figure 11 shows another possible modular arrangement.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system comprising a set of modules. There is at least a breast shield module, a pump module, a milk container module and a treatment module. In a breast pump configuration, the breast shield module, the pump module and milk container module are used without the treatment module, whereas in a treatment configuration, one or more, but not all, of the modules used in the breast pump configuration is exchanged for the treatment module.

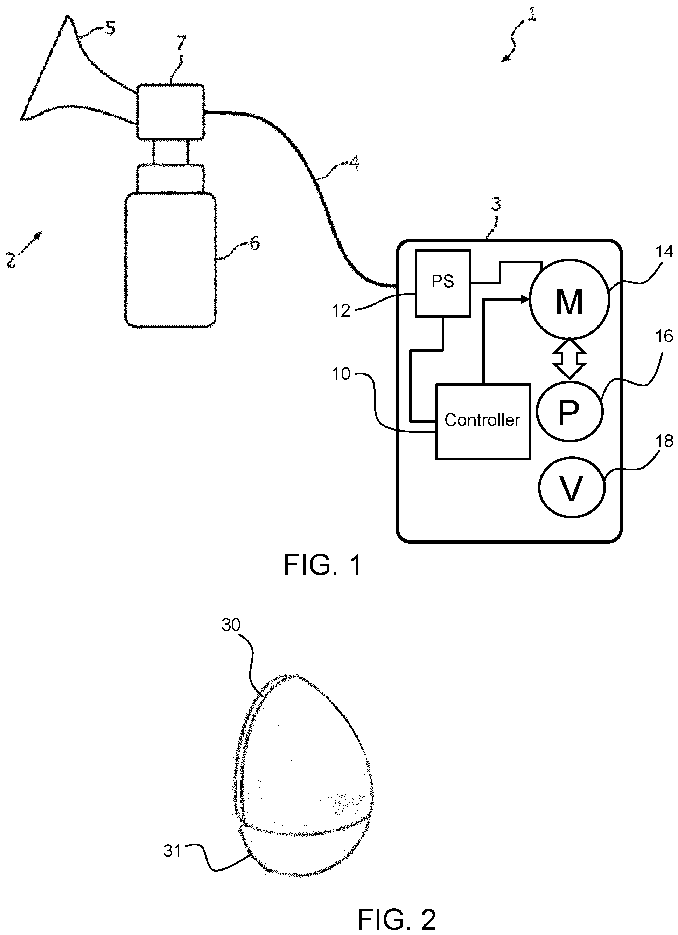

Figure 1 shows a known breast pump system 1, comprising an expression unit 2 and a drive arrangement for controlling the expression function of the breast pump.

In the example shown, the drive arrangement comprises a pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a receptacle 6 for collecting the expressed milk. The funnel 5 and the receptacle 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump 16 applies a vacuum to the membrane located in the main body 7 so that it deforms. The membrane deforms to create a vacuum in the funnel 5 which in turns applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection vessel. This enables breast pumping to be performed more discretely.

There are also other types of wearable breast pump whereby the expression unit is fixed to a breast and the pump unit and/or milk container are situated elsewhere on the body of a user.

Figure 2 shows a wearable breast pump, comprising an expression kit 30 attached to a respective milk collection vessel 31.

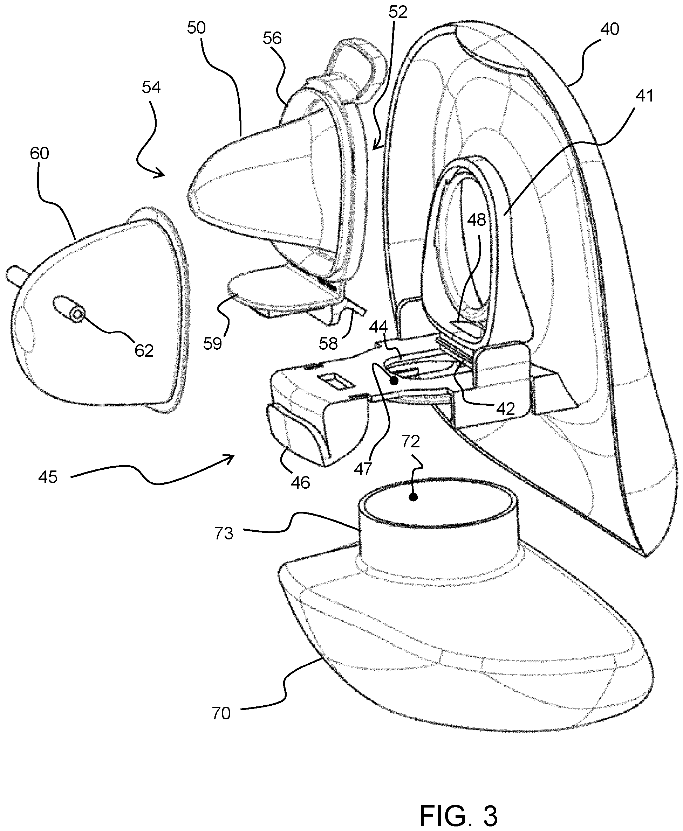

Figure 3 shows the parts of a design of breast pump that has been proposed but not yet published by the inventors. The parts shown relate to the milk flow. Figure 3 thus shows some internal parts of a breast pump, but does not show an outer housing or the vacuum source (pump) or associated control circuitry (i.e. controller, battery, valves etc.).

Figure 3 shows a breast shield 40, a diaphragm 50 formed as a flexible membrane and a vacuum cover 60 over the diaphragm 50 to define a sealed space between them. A milk collection container 70 is also shown. The cover 60 has a port 62 for coupling pressure from the vacuum source (not shown) to the sealed space between the diaphragm 50 and the cover 60.

The diaphragm 50 defines a cavity having an open end 52 for receiving (at least) a nipple of a user and a closed end 54. A connecting portion 56 in this example extends around the open end 52. It is for fitting over an opening of the breast shield 40 defined by a connection collar 41. A part of the connecting portion facing the user is for connecting the diaphragm to the collar 41 of the breast shield 40 and a part of the connecting portion 56 facing away from the user is for connecting the diaphragm to the cover 60. There is thus a connection portion 56 for connecting the diaphragm to the breast shield and/or the vacuum cover 60.

The breast shield includes an integral frame to which the other parts of the breast pump may be connected. The frame includes a release element 45 which projects forwards (i.e. away from the user) from the main body of the breast shield.

The breast shield 40 and the diaphragm form a rear assembly for fitting over the breast of a user. A front assembly fits over the rear assembly and includes the cover 60, the electrical parts of the breast pump, namely the power source (battery), pump and controller, as well as a front cover. The front assembly thus fits to, and forms a connection with, the frame, and the frame also supports the milk container. Thus, the front assembly can be removed leaving the milk container attached to the frame.

In the example shown, release element 45 extends partly or fully through a channel in the front assembly, thereby to define a release actuator 46 at the front of the breast pump. The breast shield 40 and release element 45 are formed as a single integral item.

Along the frame of the breast shield there is a rim 47 for engaging with an opening 71 of the milk container 70 formed by a top neck part 73. A rim opening 44 is formed internally of the rim 47. The milk container 70 can thus be docked reliably to the rim 47 as this a rigid part of the breast shield. Thus, the breast shield, and in particular the release element 45, acts as the support for the milk container as well as providing the support for the front assembly. A seal may be provided for sealing between the underside of the rim 47 and the opening 72 of the milk container.

The rim opening 44 has a front part (seen in Figure 3) and a rear part. The rear part of the rim opening has a top channel 48 which leads to a valve seat 42. The top channel 48 is the milk flow passage from the cavity formed by the diaphragm to the milk container 70.

The opening defined by the collar 41 of the breast shield is smaller than the opening of the open end 52 of the cavity of the diaphragm (at least at a lower area) such that milk is always caught against the outside of the opening to the breast shield and led to the container 70.

When a vacuum is applied to the sealed space between the diaphragm 50 and the cover 60 (i.e. the opposite side of the diaphragm to the cavity), the diaphragm expands compared to a rest-state when vacuum is not applied. The membrane of the diaphragm 50 touches at least one part of the breast, typically the nipple but possibly the areola and/or the breast. In the rest state, the diaphragm is non-rotationally symmetric.

Milk is expressed into the cavity in use, and is ejected towards the closed end 54 (the back) of the cavity from where it then flows to the open end and into the collection container 70.

The breast shield 40 is made of a different material to the flexible membrane of the diaphragm 50, e.g. polypropylene for the breast shield and silicone for the flexible membrane of the diaphragm. This is for ease of cleaning, for ease of use and for manufacturing reasons.

The diaphragm has a valve closure element 58, formed as in integral part of the flexible membrane, for forming part of a valve. The valve closure element may be a valve closure element of any type of valve. The valve closure element 58 is for example a flap of a flap valve. This flap valve is in the flow path from the cavity to the milk container 70 via the top channel 48.

The flap 58 extends from a bottom edge of the open end 52 of the cavity. The valve seat 42 formed by the breast shield defines the seating location for the flap valve. By integrating the movable part of a valve, in this example the flap of a flap valve, into the flexible membrane of the diaphragm, there are few parts and those few parts can be made simple to manufacture, and easy to assemble and disassemble for cleaning.

The valve may instead be formed entirely by the diaphragm.

The diaphragm also defines a lid 59. The lid 59 is for sealing the front (second) part of the rim opening 44. The purpose of the rim opening is to make the diaphragm easy to position and couple to the breast shield, while reliably placing the valve closure element in its required position. In particular, the flap 58 is fed through the front part of the opening 44 into a position where it rests (upwardly) against the valve seat 42. Thus, the flap passes through the rim opening 44 during fitting of the diaphragm to the breast shield and the lid 59 closes the rim opening 44 after fitting of the diaphragm to the breast shield. As shown more clearly below, the valve seat faces downwardly and the valve flap is biased upwardly against the valve seat.

Once in position, the lid 59 closes the front part of the opening 44 so that the only entrance to the container is via the top channel 48 and the valve. The milk collection container has a single opening 72 but it may be considered to define first and second parts; a first part beneath the valve seat 42 and a second part beneath the second part of the rim opening 44. Thus, the valve closure element provides a flow path to a milk container through a first part of the milk container opening and the lid closes a second part of the milk container opening.

The lid 59 for example comprises a gripping element for fixing the lid to the second part of the rim opening. This is for example a tab or rib or rim which is pushed through the opening 44 and engages the underside of the rim 47.

The container 70 clips to the underside of the rim 47, for example with a snap fit or with a push and rotate (bayonet) coupling.

The diaphragm thus implements the normal diaphragm function (creating a sealed volume to which the vacuum pressure can be applied), a flap valve function and a function of closing the container opening (by closing the rim opening provided to simplify the connection of the diaphragm to the breast shield). In this way, the risk of spillage from the container into the rest of the system is reduced, for example as may happen when the user is mobile. The integration of these functions by the single flexible membrane means the assembly can be easily disassembled, cleaned and re-assembled while fitting inside a small, wearable form factor, at a low cost level.

The diaphragm with its integrated valve is asymmetrical (i.e. not rotationally symmetric about a horizontal axis), so that the breast pump can be assembled only in one specific way or in a set of specific ways (if there is some rotational symmetry, e.g. rotational symmetry of order 3 for a triangular design). This has the advantage that the flexible membrane shape can be formed to touch and stimulate the nipple of the user in a predetermined way.

Furthermore, in the design shown, there are only three elements of the breast pump that come into contact with the breast milk, and need to be cleaned in a regular and thorough way, namely the breast shield 40, the membrane 50 with integrated valve 58 and lid 59 and the milk container 70.

These form the rear assembly and the container, and may thus be removed from the remainder of the breast pump as a unit. The front assembly may be removed leaving the rear assembly and the connected container, because the container is supported by the breast shield.

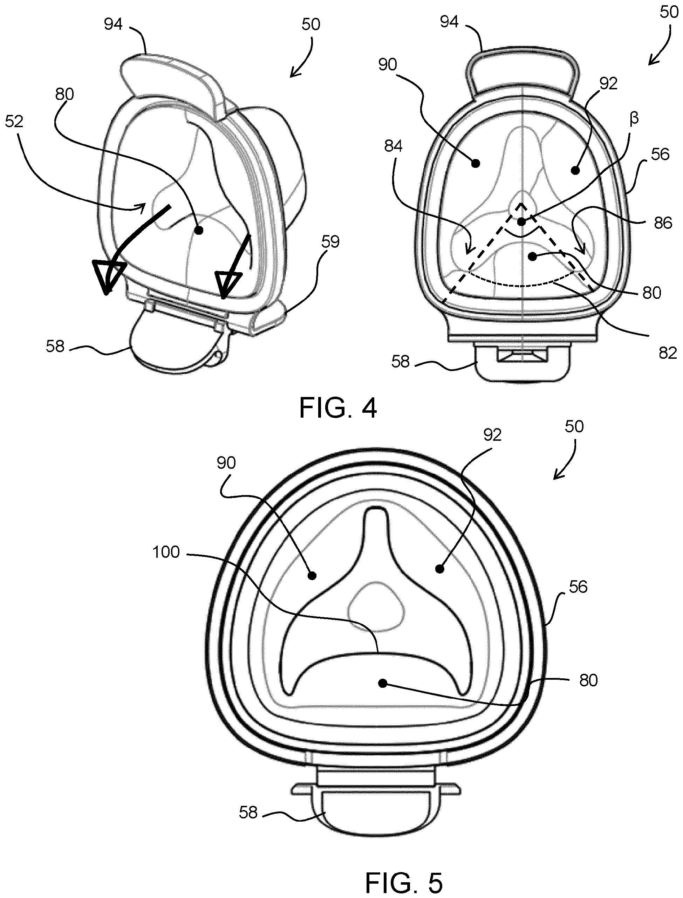

Figure 4 shows one example of a design of the diaphragm 50. The left image shows a perspective view and the right image shows a view looking into the open end 52 of the cavity.

The diaphragm comprises a radially inwardly projecting ridge 80. It extends from a base 82 of the cavity towards the center of the cavity. The ridge is preferably convex shaped so that no milk pooling can take place on the ridge. In general, the shape of the diaphragm is such that milk, in normal use, always flows towards the container.

The cavity formed by the flexible membrane thus has an internal ridge 80 extending up from a base of the cavity. The cavity is no longer rotationally symmetric such as a typical conical cavity. The ridge is a tongue-simulation ridge for simulating the tongue of a baby, and in use it presses against a lower side of the nipple, simulating the sucking action of a baby. This improves user comfort of the breast pump in that it more accurately replicates breast feeding. The ridge projects to a position which represents the position of the surface of the tongue of a baby. It may also be designed to move during application of a controlled pressure to simulate the movement of the tongue during feeding.

A first side flow path 84 is formed to a first side of the ridge 80 and a second side flow path 86 is formed to an opposite second side of the ridge 80. These flow paths facilitate the flow of milk from the closed (back) end of the diaphragm (to which the milk flow is directed) to the open (front) end 52 and then to the container. The fixed rotational orientation of the diaphragm, with the ridge projecting upwardly from the bottom, means the flow paths 84, 86 can be designed to be more effective, based on their known orientation.

The flow paths slope downwardly from the closed end of the cavity to the open end of the cavity.

The diaphragm has a default orientation in which a central axis (pointing outwardly from the user) is horizontal. In this default orientation, the top of the ridge 80 is horizontal and hence parallel with a central outward axis of the cavity. The flow paths for example slope downwardly in the range 20 to 40 degrees relative to the horizontal, i.e. relative to the ridge direction. This means that there is a range of body feeding angles of the mother which still enable the expressed milk to flow freely to the milk collection container 70.

The flow paths at the left and right of the ridge also allow for the user to angle to the left or right without leakage. Their depth at the open end of the cavity, relative to the position of the opening of the breast shield, allows the user to lean backward again without leakage. The slope of the incline mentioned above between the closed end of the cavity and the open end allows the user to lean forward without pooling of milk.

The cavity has a compact design around the nipple. The milk flow channels formed by the flow paths may for example be positioned further away from the central axis without significantly increasing the volume of the cavity, by keeping the width of the chamber around the nipple to smaller dimensions.

The ridge 80 extends most of the depth of the cavity, for example at least 50%, preferably at least 75% of the depth of the cavity (i.e. the distance between the open end and the closed end). It for example extends from the near the open end to the closed end. It may start immediately at the open end, but with a curved transition towards the top of the ridge.

The ridge 80 for example occupies an angle β around the cavity opening 52 in the range 30 to 100 degrees. The ridge for example extends from the outer edge of the open end towards the center of the open end by at least 30%, for example at least 40%, for example by at least 50% of the (vertical) radius of the open end.

Figure 4 shows second ridge 90 which extends from the first side of cavity towards the center of the cavity above the first flow path 84. There is also third ridge 92 which extends from a second side of the cavity towards the center of the cavity above the second flow path 86. The second and third ridges also extend from near the open end to the closed end. They may result in a rotationally symmetric design (with rotational symmetry of order 3) so that there are three equivalent fixed orientations with which the diaphragm may be fixed.

The second and third ridges may be designed to move when the flexible membrane deforms so that they may simulate movement the lips of the baby by providing additional contact points to the nipple in addition to the contact of the first ridge which simulates the tongue.

However, the second and third ridges may instead not move significantly when the membrane deforms, and they may instead be provided for copying the shape of a baby's lips and / or jaws rather than simulating their movements.

The use of three ridges further reduces the volume of the cavity formed between the diaphragm and the breast.

By additionally limiting the volume of the sealed space between the diaphragm and the cover, a minimum of air is needed in the system on the pump side, thus reducing dead space and increasing the efficiency of the pump system.

The design with three ridges defines a triangular arrangement of ridges. However, there may be only a single bottom ridge, or an upper ridge and a lower ridge, or more than three ridges.

As can be seen in Figure 4, the shape of the open end of the cavity, i.e. the shape formed by the path around the connecting portion 56, is also non-rotationally symmetric (as well as the internal volume of the cavity being non-rotationally symmetric). For example, it has a flatter base than top, to better match the typical shape of a baby's mouth. The shape of the open end, and indeed the diaphragm as a whole, may be left-right symmetric.

The flexibility of the flexible membrane of the diaphragm enables the contact areas to move so as to accommodate different nipple sizes.

Figure 4 additionally shows an optional positioning tab 94 extending from a top edge of the open end, i.e. extending upwardly from the connection portion 56. The positioning tab is used to simplify the connection of the diaphragm to the breast shield 40, discussed further below.

Figure 5 shows a slightly different design of the diaphragm, in which the ridge 80 has a flat top 100 to simulate the tongue shape.

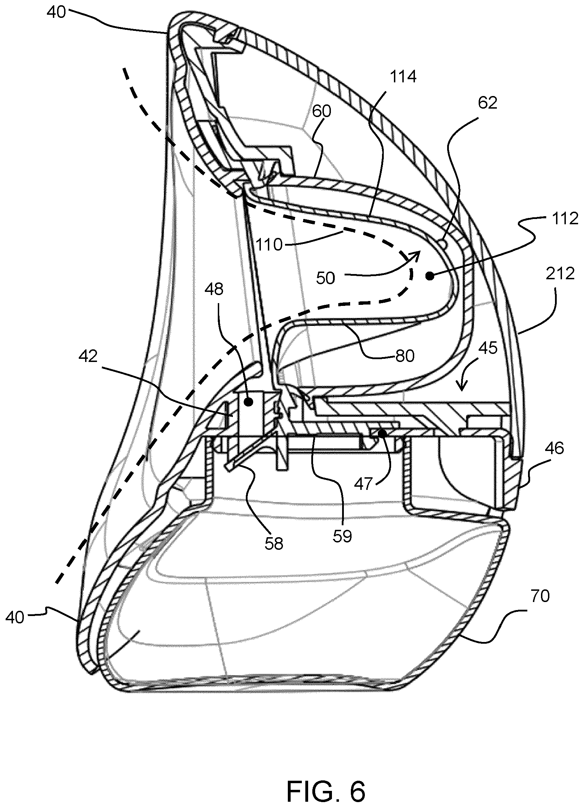

Figure 6 shows a cross section of the assembled breast pump applied to the breast. The nipple 110 is received by the cavity 112 formed by the flexible membrane 114 of the diaphragm 50.

The ridge 80 is shown as having a horizontal top edge (which may be flat or rounded in front view as shown in Figures 4 and 5).

Figure 6 also shows how the lid 59 engages under the rim 47 to hold the diaphragm in place and also to set the position of the flap 58. The outer cover 212 of the front assembly is also shown.

As mentioned above, the breast pump comprises three modules.

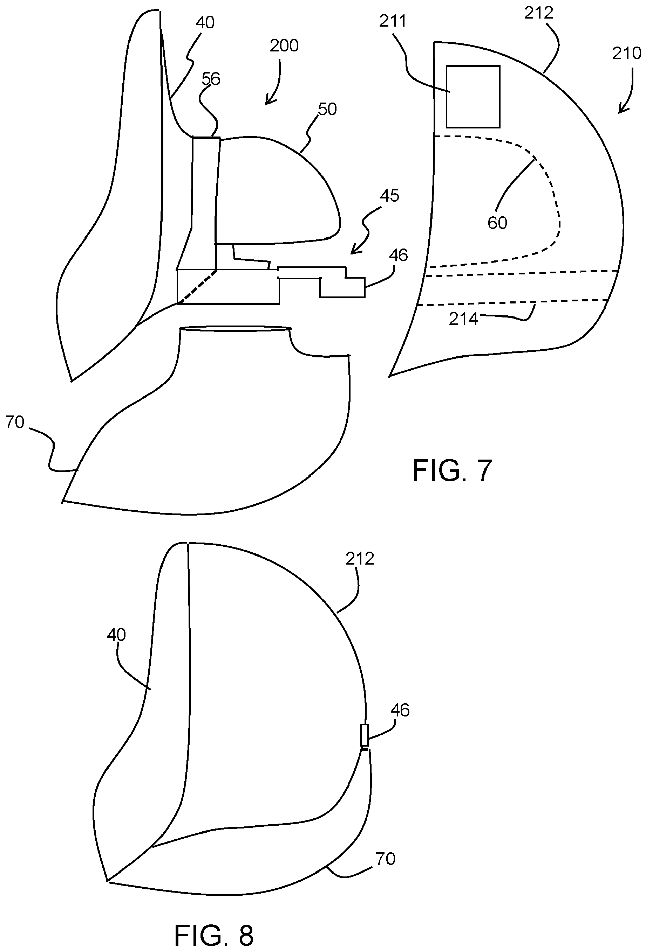

Figure 7 shows the three modules in exploded view.

A first module is a rear assembly 200 and it is for fitting over the breast of a user. It comprises the breast shield 40 (and integrated release element 45) and the diaphragm 50. A second module is a front assembly 210 for fitting over the rear assembly. It comprises the cover 60 for fitting over the diaphragm 50 to define a sealed space between them, a power source, pump and controller (and valves etc.) and a front cover 212. The power source, pump, controller and valves are shown collectively as unit 211.

The release element 45 projects forwards through a channel 214 in the front assembly to define a release actuator at a front of the front assembly. It could be exposed at the front surface or it could lie just beneath the surface, for example as a push button beneath a flexible cover.

The third module is the milk container 70, which fits beneath the rear and front assemblies.

This breast pump design is easy to assemble and disassemble for the user, by forming a rear assembly from a breast shield and a diaphragm. These are the parts (other than the container) which may come into contact with milk and therefore require regular and thorough cleaning. By forming them as an assembly, and providing the remaining parts (pump, outer housing etc.) as a front assembly, the assembly and disassembly is made simple.

The breast shield acts as support for the remaining parts of the breast pump. The breast pump can be disassembled from the front side by actuating the release actuator 46.

Figure 8 shows the three modular parts coupled together.

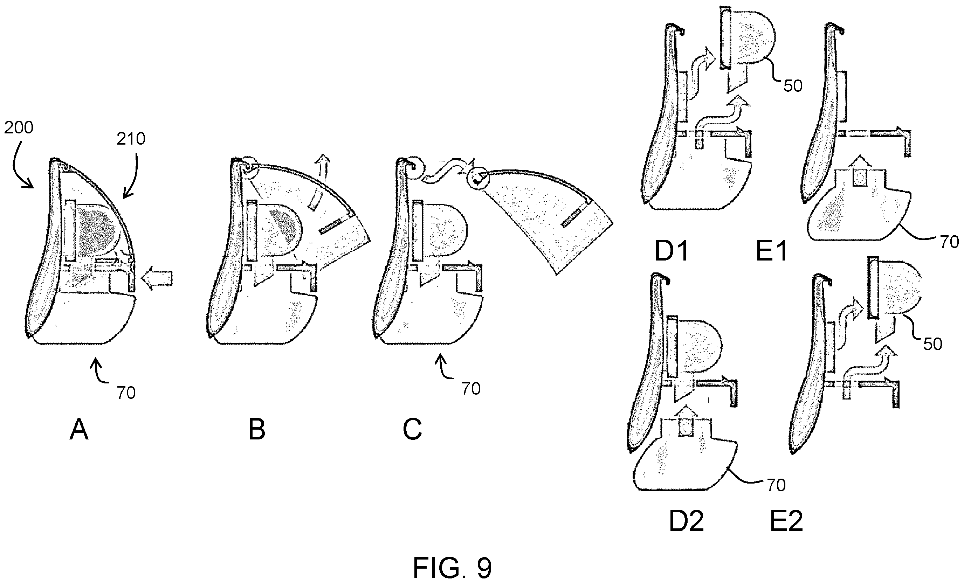

Figure 9 shows two possible disassembly routines.

In step A, the breast pump is assembled. The release actuator 46 is operated to unlatch the front and rear assemblies.

In step B, the front assembly is pivoted up. The front and rear assemblies have a releasable hinge connection at the top for this purpose.

Once opened up, the hinge connection can be released as shown in step C so that the front assembly is removed. The milk container remains attached to the breast shield of the rear assembly.

In step D1, the diaphragm 50 is removed and then in step E1 the milk container is removed.

Alternatively, in step D2, the milk container is removed and then in step E2 the diaphragm is removed.

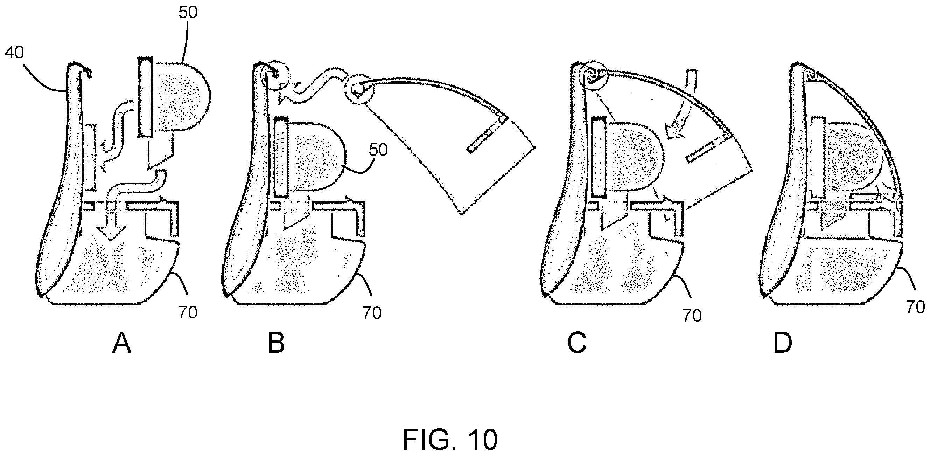

Figure 10 shows an assembly routine.

In step A the container 70 is fitted and the diaphragm 50 is fitted to the breast shield 40 (in either order) to create the complete rear assembly with container attached.

In step B the front assembly is engaged with the rear assembly at the top hinge.

In step C the front assembly is pivoted closed over the rear assembly.

In step D the front assembly clicks into engagement with the rear assembly.

The rear assembly and the front assembly may for example engage by a snap fit and the release actuator 46 releases the snap fit coupling.

The snap fit coupling may be provided on the release element 45 or on the rear assembly. Similarly, the milk collection container could be locked into place using snap fit elements or a twist and lock coupling to the rim 47.

The breast facing side of the breast shield may have a soft a cushion to improve comfort.

The coupling of the front assembly may instead use hooks to first provide an anchor to the breast shield, followed by a rotary motion lock the assemblies into place. Alternatively, the coupling may use linear sliding motion to first position elements in a guiding track, and then lock them into place.

The release element 45 may be hidden within the outer contour of the assembled breast pump system, or it can be fully or partially exposed on the outside, opposite the breast of the user when in use. As the flexible release button is integrated as part of the breast shield, the front assembly can easily be manufactured in a well-sealed and self-contained manner, for example protecting the electrics and electronics from liquids. Two-shot molding or similar techniques may be used to add materials with different characteristics, for example for release element, for sealing to the milk container or for providing a soft interface to the user.

In the examples above, the diaphragm is connected to the breast shield and then held in place by the cover. In other designs, it may instead be fitted to the cover and then the cover and diaphragm may fit to the breast shield as a unit.

The example above has three basic modules; a rear assembly, a front assembly and a milk container.

More generally, the various modules may comprise:
a breast shield module including the breast shield 40 and diaphragm 50;
a pump module 211 including at least a pump unit and electronics unit for controlling the pump; and
a milk container module 40.

The invention additionally provides a treatment module which can be exchanged for one or more of the modules, or even sub-components of the modules as defined above.

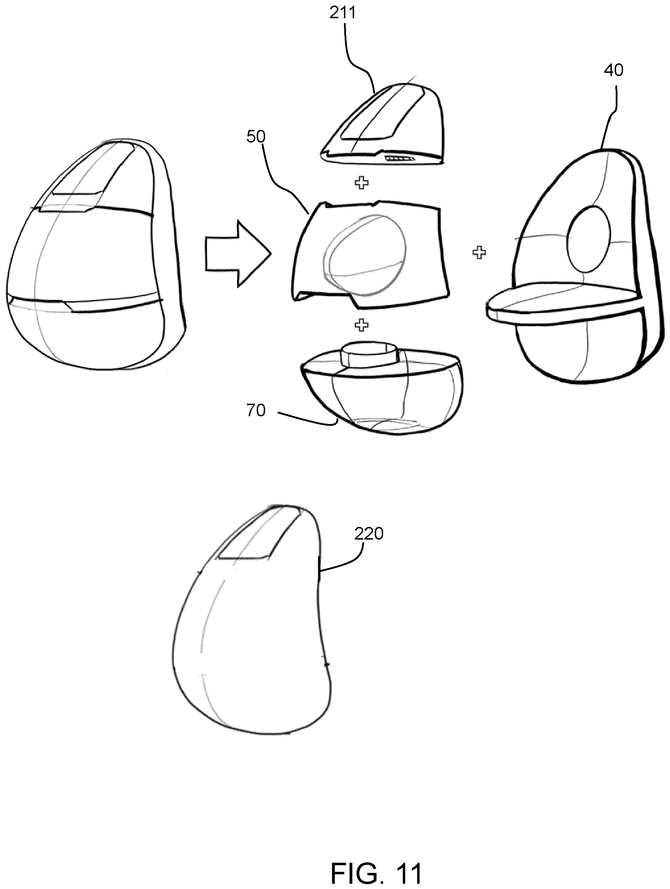

These modules are all shown in Figure 11, and with the breast shield 40 and diaphragm 50 as separate units. A treatment unit 220 is also shown, which in this example can replace the diaphragm, milk container and the pump unit.

In a breast pump configuration, a first set of the modules is used, in particular to provide the well-known breast pump operation, with a breast shield module, a pump module and a milk container module. The treatment module is not used in this configuration.

In a treatment configuration, a second set of the modules is used, including the treatment module used in the place of one or more (but not all) of the first set of modules.

In a first example, the diaphragm 50, milk container 70 and breast shield 40 can be exchanged with a treatment module in the form of a massaging module. The pump module 211 remains. By providing a suitable interface at the output of the pump module, the pump module can be used to provide the massaging module with drive energy and control functionality. Thus, the pump may be used to provide the massage motion.

In a second example, the diaphragm 50 and the milk container 70 may be exchanged with a massaging module. In this case, the breast shield 40 remains as the frame to which the other modules are attachable and detachable.

In a third example, the diaphragm 40 and the control parts of the pump module 211 are exchanged, and the massaging module has its own control module, containing for example a battery, control electronics and interface to the massaging module. The pump module may for this purpose be divided into an electronics module (such as a PCB, user interface and battery) and a separate pump unit. This arrangement may still make use of the pump unit, but with a dedicated controller. However, the massaging module may also have its own drive source such as a motor for creating a vibrational output. In such a case, the whole pump module 211 may be exchanged.

The massaging module for example induces movements in the cm range and it may provide vibrational motion.

A massage function is only one example of a possible treatment.

The treatment module may for example comprise an energy based treatment such as a light based treatment.

One example is blue light based treatment for wound care and reducing inflammation. Such a treatment may be based on a peak intensity at a wavelength in the range 400-470 nm, such as 453nm. A mean intensity may be in the range 0.02-0.2 W/cm², with a fluence in the range 10-120 J/cm² and an exposure time 900-1800s.

Another example is an infra-red based heating treatment for reducing pain or enhance milk expression. Such a treatment may be based on a peak intensity at a wavelength in the range 600-800 nm, such as 685nm. A mean intensity may be in the range 0.005-0. W/cm², with and fluence in the range 1-10 J/cm² and an exposure time in the range 60-600s.

Another example is a radio frequency based treatment for warming up the breast, or an ultrasound based treatment for warming up the breast or massaging.

Heating based treatment may be used for improving comfort and enhance milk expression and a cooling based treatment may be used for reducing pain.

In these particular examples using radiation (visible light or ultraviolet or infrared or RF) or indeed ultrasound, the treatment module comprises a radiation/ultrasound source. Other modules may be adapted to enable them to be used with the treatment module, such as a breast shield having optically transparent parts to enable the optical treatment to be applied. However, the treatment module may in that case be considered to be the module which includes the source of the radiation/ultrasound.

Similarly, for other types of treatment, there may be modules which cooperate with the main treatment module to apply the treatment, so that these other modules have a role both in the breast pump configuration and in the treatment configuration.

In further examples, topical medication may be dispersed. In such cases, the milk container module may be exchanged with a topical containing container, and the pump connection may be used to transport the topical from its container to the target area on the breast. The breast shield module (breast shield and diaphragm) may be exchanged with a pump and a unit to distribute the topical.

Sensor modules may also be added to monitor the breast or the user more generally, to monitor the application of the treatment, or to monitor the interaction between the application of the treatment and the reaction of the breast and user.

Optical sensors may be used to monitor the skin color, skin hydration, skin oiliness, blood perfusion or heart rate.

Camera-based image sensors may be used to identify cracks in the skin, redness, blood perfusion and skin color.

Capacitive and other impedance sensors may be used for skin hydration and skin oiliness.

Ultrasound sensors may identify cracks in the skin and skin thickness and detect signs of mastitis.

A temperature sensor may be used to measure the temperature of the skin.

The sensing data may be provided to an app that logs the sensing and treatment data and analyzed the data to evaluate the breast health and adapt the sensing or treatment modalities.

In an example, a module with an optical sensor such as a camera may be used to detect high blood perfusion. The signal is sent to the app where the data is processed and an inflammation indicative of nipple health issue is detected. The app then sets the appropriate blue LED in a light based treatment module for treating inflamed skin.

Various possible modules have been described above. There are of course other modules that may be added. For example, a data communication module may be provided for sending data to an external device for logging data relating to the breast pumping sessions (times, milk volumes etc.) or logging data relating to treatments, and any sensor data.

The various modules of the breast pump system may be mechanically connected to each other via means that allow the user to conveniently dissemble and reassemble all the modules. This may include methods such as magnetic attachments, locking mechanisms based on spring loading or flexing plastic features, screws etc.

Some of the modules may also be electronically connected to each other via connectors or wireless means to deliver energy or transmit data to each other. Connectors may include any variations of male and female leads that can be plugged into each other in a standard way. Wireless means may work via induction (energy) or NFC (data), Zigbee etc.

There may be different versions of modules having the same functionality. For example, different modules may perform different levels of performance, but for the same function. For example, a user may be able to choose between a lighter lower capacity battery module and a heavier, higher capacity battery module.

As explained and shown above, the modular system is for example designed to fit under a bra just like a wearable breast pump. The breast pump system may have substantially the same form factor when configured in different ways. For this purpose, some or all of the modules may have a standardized architecture in terms of the shape, size and interface towards each other, such as to define a desired overall form of the assembled system.

In the examples above, the milk container is directly attached to the remainder of the breast pump to form a wearable unit. The invention may however be applied to designs where the milk container is mounted somewhere else on the body, connected by a milk tube.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump system comprising a set of modules, the set of modules comprising:
a breast shield module (40,50) including at least a breast shield (40);
a pump module (211) including at least a pump unit;
a milk container module (70); and
a treatment module (220),
wherein the breast pump system is configurable in:
a breast pump configuration with a first set of the modules including the breast shield module, the pump module and milk container module and without the treatment module; and
a treatment configuration with a second set of the modules, in which one or more, but not all, of the first set of modules is exchanged for the treatment module.

2. The breast pump system of claim 1, wherein the pump module comprises the pump unit and an electronics unit.

3. The breast pump system of claim 1 or 2, wherein the breast shield module comprises the breast shield (40) and a diaphragm (50).

4. The breast pump system of claim 3, wherein the diaphragm is formed of a flexible membrane (114) and comprises a cavity (112) having an open end (52) for receiving a nipple of a user and a closed end (54), wherein the diaphragm fits over an opening in the breast shield (40).

5. The breast pump system of claim 3 or 4, wherein the breast shield and the diaphragm form a rear assembly (200) and the pump module is part of a front assembly that fits over the rear assembly, wherein the front assembly (210) further comprises a cover (60) for fitting over the diaphragm (50) to define a sealed space between them.

6. The breast pump system of any one of claims 1 to 5, wherein the second set of the modules, for the treatment configuration, comprises the pump module and the treatment module without the milk container module and without the breast shield module.

7. The breast pump system of claim 3, 4 or 5, wherein the second set of the modules, for the treatment configuration, comprises the pump module, the treatment module and the breast shield, without the milk container module and without the diaphragm.

8. The breast pump system of any one of claims 1 to 7, wherein the set of modules includes a treatment control module, wherein the second set of the modules, for the treatment configuration, comprises at least the treatment module and the treatment control module.

9. The breast pump system of any one of claims 1 to 8, wherein the treatment module comprises:
a massage unit; or
a light delivery unit for phototherapy

10. The breast pump system of claim 9, wherein the treatment module comprises a light delivery unit for delivering light with a peak intensity at a wavelength in the range 400nm to 470nm; or infrared light with a peak intensity at a wavelength in the range 600nm to 800nm.

11. The breast pump system of any one of claims 1 to 8, wherein the treatment module comprises:
an RF delivery system or an ultrasound delivery system or a cooling system such as using a Peltier cooling element; or
a system for delivering topical medication, wherein the set of modules further comprises a topical medication storage container.

12. The breast pump system of any one of claims 1 to 11, wherein the set of modules further comprises a treatment monitoring sensor module to monitor the application of the treatment or to monitor the interaction between the application of the treatment and the reaction of the breast and user, and the second set of the modules, for the treatment configuration, includes the treatment monitoring sensor module.

13. The breast pump system of claim 12, wherein the treatment monitoring sensor system comprises one or more of:
an optical sensor for monitoring skin color, skin hydration, skin oiliness, blood perfusion or heart rate;
an impedance or capacitance sensor for monitoring skin hydration or skin oiliness;
an ultrasound sensor for monitoring skin condition; or
a temperature sensor for measuring skin temperature.

14. A treatment module for applying a treatment to a breast, adapted to be exchangeable with one or more, but not all, of the modules of the first set of the modules of the breast pump system of any one of claims 1 to 13, thereby to result in the second set of modules.

15. A breast pump module adapted to form one of the first set of modules of the breast pump system of any one of claims 1 to 13, wherein said breast pump module is adapted to cooperate with a treatment module to enable application of treatment to a breast, when the breast pump system is in the treatment configuration.
